# EUROPÄISCHE PATENTSCHRIFT

(11) **EP 1 374 925 B1**
(45) Veröffentlichungstag und Bekanntmachung des Hinweises auf die Patenterteilung: **26.04.2006**
(21) Anmeldenummer: 02014113.1
(22) Anmeldetag: 24.06.2002
(51) Int. Cl.: A61M 1/00, A61M 3/02

(54) **Vorrichtung zum Spülen eines Körperhohlraumes mit einer Flüssigkeit**
Device for the irrigation of a body cavity
Appareil pour l'irrigation d'une cavité corporelle

(43) Veröffentlichungstag der Anmeldung: 02.01.2004
(73) Patentinhaber: Storz Endoskop Produktions GmbH, 78532 Tuttlingen (DE)
(72) Erfinder: Novak, Pavel, 8207 Schaffhausen (CH)
(74) Vertreter: Heuckeroth, Volker

(56) Entgegenhaltungen:
- WO-A-00/78372
- DE-A- 4 219 890
- US-A- 4 261 360
- US-A- 4 650 462
- US-A- 5 685 821

## Beschreibung

Die Erfindung betrifft eine Vorrichtung zum Spülen eines Körperhohlraumes mit einer Flüssigkeit, mit einer Spülpumpe und einer dieser zugeordneten, zum Körperhohlraum hin führbaren Spülleitung zum Zuführen der Flüssigkeit in den Körperhohlraum, und mit zumindest einer von dem Körperhohlraum wegführbaren Saugleitung und einer dieser zugeordneten Saugpumpe zum Absaugen der Flüssigkeit von dem Körperhohlraum, und mit einer Steuerschaltung, die die Spülpumpe und die Saugpumpe derart steuert, daß die Flüssigkeit im wesentlichen mit einem vorbestimmten Soll-Druck und einer vorbestimmten Soll-Flußrate durch den Körperhohlraum tritt, wobei die Steuerschaltung die Spülpumpe in Abhängigkeit der Soll-Flußrate steuert, während sie die Saugpumpe in Abhängigkeit des Soll-Drucks steuert.

Eine solche Vorrichtung ist aus dem Dokument US 5,685,821 bekannt.

Eine Vorrichtung der genannten Art wird für die endoskopische Spülung von Körperhohlräumen, beispielsweise der Harnblase, der Gebärmutter, oder auch von Gelenkkapseln verwendet.

Die aus dem oben genannten Dokument US 5,685,821 bekannte Vorrichtung weist eine Spülpumpe auf, die durch die Steuerschaltung so gesteuert wird, daß sie eine vorbestimmte Soll-Flußrate in den Körperhohlraum bereitstellt, während die Saugpumpe so gesteuert ist, daß sie durch eine variable Drehgeschwindigkeit den Druck im Körperhohlraum steuert. Dadurch soll der Druck im Körperhohlraum mit größerer Genauigkeit kontrolliert werden können, indem der Druckabfall kompensiert wird, der durch den Abschnitt der Spülleitung zwischen dem Drucksensor und dem Körperhohlraum auftritt. Mit einer festen Spülflußrate kann der Druckabfall durch die Spülleitung genau bestimmt werden, und daher kann das Ausgangssignal des Drucksensors, der an dem Auslaß der Spülpumpe angeordnet ist, genauer den tatsächlichen Druck der Spülflüssigkeit in dem Körperhohlraum anzeigen.

Aus dem Dokument DE 42 19 890 A1 ist eine Vorrichtung zum Spülen von Körperhöhlen bekannt, die eine Spülpumpe und eine Saugpumpe aufweist, die jeweils mit einem Drucksensor ausgestattet sind und vorzugsweise als Schlauchrollenpumpen ausgeführt sind. Die Spülpumpe und die Saugpumpe sind miteinander über eine Steuerleitung verbunden und arbeiten in einer Art Master-/Slave-Betrieb. Es wird nur die Spülpumpe eingestellt und vom Operateur bedient. Die Steuerung der Saugpumpe erfolgt automatisch auf Grund der Meßwerte beider Drucksensoren in der Spül- und Absaugleitung, sowie auf Grund der eingestellten Drehzahl der Spülpumpe.

Eine aus dem Dokument US 4,902,277 bekannte Vorrichtung wird speziell in der Arthroskopie bei einer endoskopischen Resektion eines Gelenks verwendet. Dabei wird eine physiologische Flüssigkeit mittels einer Spülpumpe durch eine Spülleitung in das Gelenk gepumpt. Über eine Saugleitung wird dann die Flüssigkeit mittels einer Saugpumpe aus dem Gelenk wieder abgesaugt.

Bei einer derartigen endoskopischen Spülung von Hohlorganen ist es wünschenswert, daß der Druck und die Flußrate der Spülflüssigkeit im Körperhohlraum unabhängig voneinander eingestellt werden können. Unter der Flußrate wird dabei der Flüssigkeitsdurchsatz durch den Körperhohlraum pro Zeiteinheit verstanden.

Bei der zuvor genannten bekannten Vorrichtung ist die Spülpumpe eine Verdrängerpumpe, die in Abhängigkeit von dem Ist-Druck in der Spülleitung oder im Körperhohlraum, der mittels eines Drucksensors gemessen wird, gesteuert wird, um einen vorbestimmten Soll-Druck in dem Körperhohlraum aufrecht zu erhalten. In Abhängigkeit des vom Drucksensor gemessenen Ist-Druckes wird die Drehzahl des Motors der Spülpumpe erhöht oder erniedrigt, um den Soll-Druck im Körperhohlraum aufrecht zu erhalten.

Die Saugpumpe dieser bekannten Vorrichtung ist ebenfalls eine Verdrängerpumpe, die in Abhängigkeit einer vorbestimmten Soll-Flußrate gesteuert ist. Dabei können zwei Soll-Flußraten eingestellt werden, in deren Abhängigkeit die Saugpumpe gesteuert ist. Die Saugpumpe hält die jeweils eingestellte vorbestimmte Soll-Flußrate aufrecht. Die höhere Soll-Flußrate kann beispielsweise eingestellt werden, wenn in dem Körperhohlraum zusätzlich ein Arbeitsinstrument, beispielsweise ein Resektionsinstrument, in Betrieb genommen wird. Die niedrigere Soll-Flußrate kann beispielsweise dann eingestellt werden, wenn lediglich der Körperhohlraum gespült werden soll, ohne daß ein Arbeitsinstrument im Körperhohlraum in Betrieb ist.

Die Verwendung einer Verdrängerpumpe, insbesondere einer Rollenpumpe, hat den Vorteil, daß die von der Pumpe erzeugte Flußrate der Drehzahl der Verdrängerpumpe weitestgehend proportional ist, vor allem dann, wenn der von der Spülpumpe aufrechterhalte Druck konstant ist.

Ein Nachteil dieser bekannten Vorrichtung tritt dann zutage, wenn in der Saugleitung eine Obstruktion, die beispielsweise durch Gewebestücke verursacht wird, auftritt. Bei einer Obstruktion in der Saugleitung steigt der Ist-Druck im Körperhohlraum über den Soll-Druck mit der Folge, daß bei der bekannten Vorrichtung die Spülpumpe dann so gesteuert wird, daß ihre Drehzahl abnimmt, um den Soll-Druck aufrechtzuerhalten. Die Saugpumpe, die auf die vorbestimmte Soll-Flußrate eingestellt ist, arbeitet dagegen mit unveränderter Drehzahl weiter. Dies hat zur Folge, daß die Obstruktion von der Saugpumpe unter Umständen nicht durch Absaugung beseitigt werden kann. Dies kann zu einer Störung und Unterbrechung des Spülvorganges und damit des medizinischen Eingriffs führen.

Demgegenüber ist aus der EP 0 529 902 A2 eine Vorrichtung zum Spülen eines Körperhohlraums bekannt, bei der die Spülpumpe eine Zentrifugalpumpe ist, die in Abhängigkeit eines vorbestimmten Soll-Druckes im Körperhohlraum bzw. in der Spülleitung gesteuert ist, während die Saugpumpe eine Verdrängerpumpe in Form einer Zahnradpumpe ist. Im Unterschied zu einer Verdrängerpumpe weist eine Zentrifugalpumpe die Eigenschaft auf, daß die erzeugte Flußrate keine eindeutige Abhängigkeit von der Drehzahl der Zentrifugalpumpe aufweist, sondern zusätzlich vom Ist-Druck abhängt. Bei dieser bekannten Vorrichtung wird die Flußrate entsprechend mit einem steuerbaren Schlauchquetschventil geregelt.

Diese bekannte Vorrichtung hat den Nachteil, daß die Spülpumpe, die als Zentrifugalpumpe ausgebildet ist, nicht dichtend ist, so daß im Fall, daß das Quetschventil nicht geschlossen ist und beide Pumpen nicht aktiviert sind, die Flüssigkeit auf Grund des Gefälles frei durchlaufen kann. Ein weiterer Nachteil dieser bekannten Vorrichtung besteht wie oben erwähnt darin, daß durch die Verwendung einer Zentrifugalpumpe als Spülpumpe die Fluß-Rate nicht genau vorgegeben werden kann, weil weder die Spül- noch die Saugpumpe eine einfache Zuordnung der Flußrate beispielsweise in Abhängigkeit der Drehzahl der Pumpen erlauben. Bei dieser bekannten Vorrichtung ist die Ist-Flußrate sehr von der Druckdifferenz im System abhängig.

Der Erfindung liegt die Aufgabe zugrunde, eine Vorrichtung der eingangs genannten Art bereitzustellen, mit der auf andere Weise als bei den bekannten Vorrichtungen der Druck und die Flußrate möglichst unabhängig voneinander gesteuert werden können.

Erfindungsgemäß wird diese Aufgabe hinsichtlich der eingangs genannten Vorrichtung dadurch gelöst, daß die Steuerschaltung desweiteren derart ausgebildet ist, daß sie die Drehzahl der Saugpumpe als Gebersignal verwendet und in Abhängigkeit dieses Gebersignals die Spülpumpe steuert.

Bei der erfindungsgemäßen Vorrichtung wird die Spülpumpe nicht in Abhängigkeit des Soll-Drucks gesteuert, sondern mit der Spülpumpe wird die vorbestimmte Soll-Flußrate eingestellt. Dagegen wird mittels der Saugpumpe der vorbestimmte Soll-Druck eingestellt, das heißt die Saugpumpe ist auf den vorbestimmten Soll-Druck gesteuert. Dies hat den Vorteil, daß Obstruktionen in der Saugleitung, die zu einer Erhöhung des Ist-Druckes über den Soll-Druck hinaus führen, durch eine sich automatisch einstellende höhere Saugleistung der Saugpumpe durch Absaugung beseitigt werden können. Die aus der US-4,902,277 bekannte Vorrichtung würde dagegen nur auf eine Obstruktion in der Spülleitung durch eine Verminderung der Drehzahl der Spülpumpe reagieren, wodurch die Obstruktion jedoch nicht beseitigt wird. Eine Obstruktion in der Spülleitung hat daneben in der Praxis so gut wie keine Relevanz, da in der frischen Spülflüssigkeit aus dem Vorratsgefäß keine Obstruktionen zu befürchten sind.

Erfindungsgemäß verwendet die Steuerschaltung die Drehzahl der Saugpumpe als Gebersignal und steuert in Abhängigkeit dieses Gebersignals die Spülpumpe.

Diese Ausgestaltung ist insbesondere dann von Vorteil, wenn als Spülpumpe eine Zahnradpumpe verwendet wird, bei der die Flußrate nicht eindeutig mit der Drehzahl der Pumpe korreliert ist, wie es in der Regel bei einer Rollenpumpe der Fall ist. Bei einer Zahnradpumpe hängt die Flußrate sowohl von dem Saugdruck als auch von dem Spüldruck ab. Aus diesem Grund ist es nicht möglich, die Flußrate allein mittels der Drehzahl der Zahnradpumpe zu steuern, sondern es bedarf einer Regelung. Eine solche Regelung der Drehzahl der Spülpumpe kann auf vorstehend genannte Weise in Abhängigkeit der Drehzahl der vorzugsweise als Rollenpumpe ausgebildeten Saugpumpe einfach erzielt werden. Auch ist diese Ausgestaltung geeignet, um die Sicherheit der Vorrichtung für den Patienten zu verbessern.

Dazu ist es in einer bevorzugten Ausgestaltung vorgesehen, daß der Spülleitung ein Drucksensor zur Erfassung des Ist-Druckes zugeordnet ist, und daß die Steuerschaltung die Saugpumpe in Abhängigkeit des vom Drucksensor erfaßten Ist-Druckes zur Aufrechterhaltung des Soll-Druckes regelt.

Hierbei ist von Vorteil, daß der Soll-Druck besonders exakt aufrechterhalten werden kann, in dem die Saugleistung der Saugpumpe in Abhängigkeit von dem erfaßten Ist-Druck erhöht bzw. erniedrigt wird, um den vorbestimmten Soll-Druck im Körperhohlraum aufrechtzuerhalten.

In einer weiteren bevorzugten Ausgestaltung steuert die Steuerschaltung die Spülpumpe in Abhängigkeit der Saugleistung der Saugpumpe derart, daß bei einer Erhöhung der Saugleistung der Saugpumpe, zumindest wenn diese über einen vorbestimmten Schwellenwert steigt, die Leistung der Spülpumpe reduziert wird.

Diese Maßnahme ist bezüglich der Sicherheit der Vorrichtung für den Patienten besonders vorteilhaft. Im Fall einer Obstruktion, bei der der Ist-Druck im Körperhohlraum über einen Schwellenwert steigt und entsprechend die Saugpumpe ihre Saugleistung stark erhöht, um den vorbestimmten Soll-Druck aufrechtzuerhalten, aber die Obstruktion derart ist, daß sie trotz Erhöhung der Saugleistung nicht beseitigt werden kann, wird die Leistung der Spülpumpe erniedrigt oder gar auf null reduziert, um einen zu hohen Druck im Körperhohlraum und damit eine Gefahr für den Patienten zu vermeiden. Es wird somit vermieden, daß bei einer nicht durch Absaugung beseitigbaren Obstruktion noch weiter Spülflüssigkeit in den Körperhohlraum gepumpt wird.

In einer weiteren bevorzugten Ausgestaltung sind die Spülpumpe und/oder die Saugpumpe jeweils Verdrängerpumpen.

Die Verwendung von Verdrängerpumpen hat den Vorteil, daß eine Steuerung dieser Pumpen auf einfache Weise über die Drehzahl erfolgen kann, wobei der weitere Vorteil darin besteht, daß der erzeugte Durchsatz bzw. die Flußrate eine im wesentlichen eindeutige Abhängigkeit von der Drehzahl der Pumpe aufweist.

In diesem Zusammenhang ist es bevorzugt, wenn die Spülpumpe eine Rollenpumpe oder Zahnradpumpe ist.

Eine Rollenpumpe ist eine Verdrängerpumpe, bei der eine Mehrzahl von Rollen entlang eines flexiblen Leitungsabschnitts bewegt werden, wodurch die Flüssigkeitsleitung peristaltisch im Querschnitt verengt wird. Die Verwendung einer Rollenpumpe ist deswegen besonders bevorzugt, weil bei einer Rollenpumpe die Flußrate eindeutig mit der Drehzahl der Rollenpumpe korreliert ist.

Die Verwendung einer Zahnradpumpe als Spülpumpe hat dagegen den Vorteil, daß die Spülflüssigkeit im wesentlichen pulsationsfrei in den Körperhohlraum zugeführt wird.

Weiterhin ist es bevorzugt, wenn die Saugpumpe eine Rollenpumpe ist.

Obwohl mit einer Rollenpumpe kein pulsationsfreier Fluß erzeugt werden kann, was für die Saugpumpe auch nicht unbedingt erforderlich ist, hat eine Rollenpumpe den Vorteil eines eindeutigen Zusammenhangs zwischen der Flußrate und der Drehzahl der Pumpe und eines konstruktiv besonders einfachen Aufbaus.

In alternativen Ausgestaltungen kann es auch bevorzugt sein, wenn die Spülpumpe in Form einer Zentrifugalpumpe ausgestaltet ist. Um hier vorteilhafterweise einen Zusammenhang zwischen der Flußrate und der Drehzahl zu erhalten, wird vorab für einen bestimmten Typ einer zentrifugalpumpe die Ist-Korrelation zwischen der Flußrate und der Drehzahl ermittelt, auf die die Steuerschaltung dann zur Steuerung der Spülpumpe zurückgreift, um die vorbestimmte Soll-Flußrate einzustellen bzw. aufrechtzuerhalten.

Im Zusammenhang mit einer der zuvor genannten Ausgestaltungen, wonach die Steuerschaltung die Spülpumpe in Abhängigkeit der Saugleistung der Saugpumpe derart steuert, daß bei einer Erhöhung der Saugleistung, zumindest wenn diese über einen vorbestimmten Schwellenwert steigt, die Leistung der Spülpumpe reduziert wird, ist in einer weiteren bevorzugten Ausgestaltung vorgesehen, daß die Steuerschaltung die Spülpumpe zumindest bei Überschreiten des Schwellenwertes in Abhängigkeit der Drehzahl der Saugpumpe steuert, derart, daß die Drehzahl der Spülpumpe vermindert wird, wenn die Drehzahl der Saugpumpe steigt.

Auch hierbei besteht der Vorteil in einer besonders einfach zu realisierenden Steuerung der Spülpumpe in Abhängigkeit von der Saugleistung der Saugpumpe, die den gewünschten Effekt bewirkt, daß ein Gefahrenzustand durch eine ständige Druckzunahme im Körperhohlraum vermieden wird.

In einer weiteren vorteilhaften und bevorzugten Ausgestaltung kann die Steuerschaltung die Spülpumpe auch in Abhängigkeit der Differenz der Drehzahlen von Spülpumpe und Saugpumpe steuern, zumindest wenn diese Differenz einen vorbestimmten Wert übersteigt.

In einer weiteren bevorzugten Ausgestaltung steuert die Steuerschaltung die Spülpumpe in Abhängigkeit zumindest zweier unterschiedlicher Soll-Flußraten.

In dieser Ausgestaltung können mit der Spülpumpe zumindest zwei unterschiedliche Soll-Flußraten eingestellt werden. Bei einem chirurgischen Eingriff, beispielsweise einem endoskopischen Eingriff, kann vorteilhafterweise der Flüssigkeitsdurchsatz durch den Körperhohlraum verstärkt werden, beispielsweise wenn in dem Körperhohlraum Gewebe abgetragen wird, so daß Blut und Gewebeteile durch den erhöhten Spülfluß effizienter abgeführt werden können.

In diesem Zusammenhang ist es bevorzugt, wenn die Steuerschaltung die Spülpumpe zwischen den zumindest zwei Soll-Flußraten in Abhängigkeit des Betriebszustandes eines mit der Steuerschaltung verbundenen Arbeitsinstruments umschaltet.

Hierbei ist von Vorteil, daß die Umschaltung zwischen den zumindest zwei Soll-Flußraten automatisch erfolgt, wenn das Arbeitsinstrument eingeschaltet oder ausgeschaltet wird, so daß der Arzt die Umschaltung nicht selbst vornehmen muß und dadurch von seiner operativen Tätigkeit nicht abgelenkt wird.

In einer weiteren bevorzugten Ausgestaltung führen zumindest zwei parallele Saugleitungen von dem Körperhohlraum weg, wobei die Saugpumpe beiden Saugleitungen zugeordnet ist, wobei ferner eine Saugleitung mit einem Arbeitsinstrument verbunden ist, und wobei zwischen den beiden Saugleitungen in Abhängigkeit des Betriebszustandes des Instruments umgeschaltet werden kann.

In diesem Fall ist das für einen operativen Eingriff verwendete Arbeitsinstrument, beispielsweise ein Resektionsinstrument, gleichzeitig als Sauginstrument ausgestaltet, und beim Aktivieren des Arbeitsinstruments wird dann die Flüssigkeit und abgetragenes Gewebe über das Arbeitsinstrument abgesaugt, wobei auch in diesem Fall das Umschalten zwischen den beiden Saugleitungen automatisch erfolgt, ohne daß der Arzt die Umschaltung selbst vornehmen muß.

In einer weiteren bevorzugten Ausgestaltung ermittelt die Steuerschaltung die Differenz zwischen den Drehzahlen der Saugpumpe und der Spülpumpe.

Diese Maßnahme ist insbesondere dann von Vorteil, wenn als Spülpumpe und als Saugpumpe jeweils eine Rollenpumpe verwendet wird, bei denen der Zusammenhang zwischen der Flußrate und der Drehzahl eindeutig ist. In diesem Fall hat die vorgenannte Ausgestaltung nämlich den Vorteil, daß der Flüssigkeitsverlust im Körperhohlraum, beispielsweise durch Eintreten von Flüssigkeit in die Blutbahn, durch Subtraktion der beiden Drehzahlen der beiden Pumpen relativ gut erfaßt werden kann.

Weitere Vorteile und Merkmale ergeben sich aus der nachfolgenden Beschreibung und beigefügten Zeichnung.

Es versteht sich, daß die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hier noch näher beschrieben. Es zeigen:
- Fig. 1: ein schematisches Schaltbild einer Vorrichtung zum Spülen eines Körperhohlraums gemäß einem ersten Ausführungsbeispiel;
- Fig. 2: die Vorrichtung in Fig. 1 mit weiteren Einzelheiten der Steuerschaltung der Vorrichtung;
- Fig. 3: den Kurvenverlauf einer alternativen Steuerungsabhängigkeit zwischen Spülpumpe und Saugpumpe der Vorrichtung in Fig. 1; und
- Fig. 4: ein Schaltbild einer Vorrichtung gemäß einem weiteren Ausführungsbeispiel.

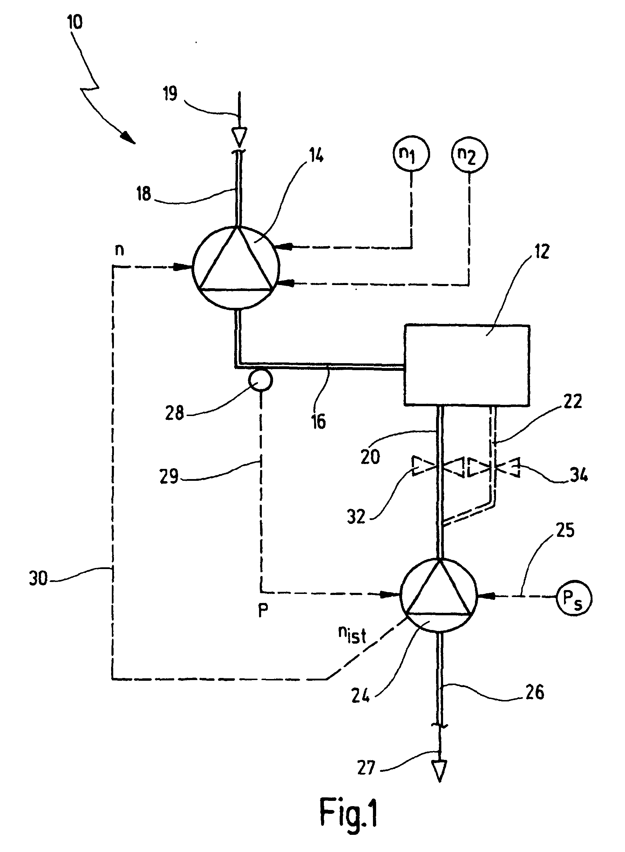

In Fig. 1 ist eine mit dem allgemeinen Bezugszeichen 10 versehene Vorrichtung zum Spülen eines Körperhohlraums 12 mit einer Flüssigkeit schematisch dargestellt. Die Flüssigkeit ist beispielsweise eine physiologische Flüssigkeit.

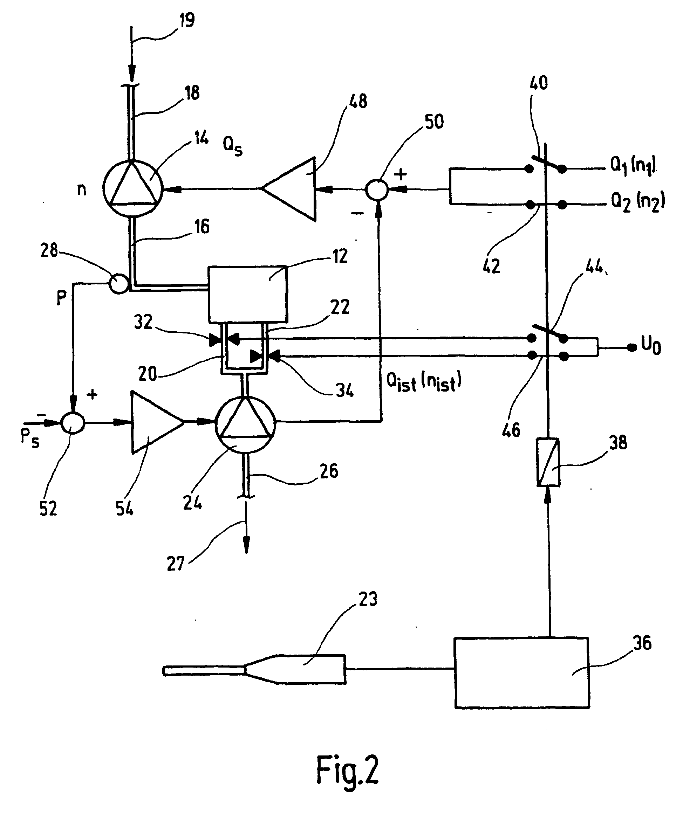

In Fig. 2 ist die Vorrichtung 10 mit einer schematischen Darstellung einer zugehörigen Steuerschaltung dargestellt.

Die Vorrichtung 10 wird beispielsweise zur endoskopischen Spülung der Harnblase, Gebärmutter oder im Rahmen der Arthroskopie zur Spülung von Gelenken, beispielsweise des Knies, verwendet.

Die Vorrichtung 10 weist eine Spülpumpe 14 auf, die mit einer Spülleitung 16 verbunden ist, die zum Körperhohlraum 12 hin führt. Die Flüssigkeit wird über eine Leitung 18 gemäß einem Pfeil 19 von einem Flüssigkeitsreservoir entnommen.

Stromabwärts von dem Körperhohlraum 12 führt eine erste Saugleitung 20 von dem Körperhohlraum 12 zu einer Saugpumpe 24. Eine zweite Saugleitung 22, die der ersten Saugleitung 20 parallel geschaltet ist, führt ebenfalls von dem Körperhohlraum 12 zu der Saugpumpe 24. Die erste Saugleitung 20 ist mit einem nicht näher dargestellten Spülinstrument verbunden, daß in den Körperhohlraum 12 einsetzbar ist. Die zweite Saugleitung 22 ist mit einem in Fig. 2 schematisch dargestellten Arbeitsinstrument 23, beispielsweise einem Resektionsinstrument, verbunden, daß sowohl zum Saugen als auch zum Durchführen eines Eingriffs in dem Körperhohlraum 12, beispielsweise zum Abtragen von Gewebe, verwendet wird.

Mittels der Saugpumpe 24 wird über die erste Saugleitung 20 oder über die zweite Saugleitung 22 die Flüssigkeit aus dem Körperhohlraum 12 nebst Körperflüssigkeiten, wie Blut, Sekrete und Gewebestücken, abgesaugt. Die abgesaugte Flüssigkeit wird über eine Leitung 26 gemäß einem Pfeil 27 zu einem nicht dargestellten Auffangbehälter abgeführt.

Der Spülleitung 16 ist weiterhin ein Drucksensor 28 zugeordnet, der in der Lage ist, den Ist-Druck p in der Spülleitung 16 bzw. in dem Körperhohlraum 12 zu erfassen. Die Erfassung des Ist-Druckes im Körperhohlraum 12 kann beispielsweise durch Berechnung des Ist-Druckes in der Spülleitung 16 bei bekanntem Strömungswiderstand der Spülleitung 16 erfolgen.

Die Spülpumpe 14 ist eine Verdrängerpumpe, und im vorliegenden Fall ist die Spülpumpe 14 eine Rollenpumpe oder Zahnradpumpe. Eine Rollenpumpe ist beispielsweise in dem Dokument EP 0 448 909 B1 beschrieben. Eine Rollenpumpe ist eine peristaltische Pumpe, bei der eine Mehrzahl von Rollen auf einer Kreisscheibe angeordnet sind, die sich um ihre Mittelachse dreht. Beim Drehen der Kreisscheibe laufen die Rollen entlang eines Schlauchstückes der Saugleitung 26 und verändern dabei peristaltisch den Querschnitt dieses Leitungsstückes. Eine medizinische Zahnradpumpe ist beispielsweise in dem Dokument 197 25 462 A1 derselben Anmelderin beschrieben, auf das hier zur beispielhaften Erläuterung des Aufbaus und der Funktion einer Zahnradpumpe verwiesen wird.

Die Vorrichtung 10 weist eine in Fig. 2 dargestellte Steuerschaltung auf, die die Spülpumpe 14 in Abhängigkeit einer vorbestimmten Soll-Flußrate Qₛ steuert, die in dem Körperhohlraum 12 herrschen soll. Die vorbestimmte Soll-Flußrate Qₛ kann einfach durch Einstellung einer bestimmten Drehzahl n der Spülpumpe 14 eingestellt werden, insbesondere wenn diese eine Rollenpumpe ist.

Die Steuerschaltung steuert die Spülpumpe 14 jedoch nicht nur in Abhängigkeit einer vorbestimmten Soll-Flußrate, sondern in Abhängigkeit von zwei Soll-Flußraten Q₁ und Q₂, einer höheren und einer niedrigeren, denen entsprechend zwei Drehzahlen n₁ (höhere Soll-Flußrate) und n₂ (kleinere Soll-Flußrate) zugeordnet sind.

Die Saugpumpe 24 wird von der Steuerschaltung dagegen in Abhängigkeit eines vorbestimmten Soll-Drucks pₛ (unterbrochene Linie 25 in Fig.1) gesteuert, der in dem Körperhohlraum 12 herrschen soll. Die Saugpumpe 24 wird von der Steuerschaltung zur Aufrechterhaltung des vorbestimmten Soll-Druckes pₛ in dem Körperhohlraum 12 ferner in Abhängigkeit von dem vom Drucksensor 28 erfaßten Ist-Druck p geregelt.

Die Saugpumpe 24 ist ebenfalls eine Verdrängerpumpe, die im vorliegenden Fall eine Rollenpumpe ist, aber auch eine Zahnradpumpe sein kann.

Wie bereits erwähnt, ist die Spülpumpe 14 in Abhängigkeit zumindest zweier vorbestimmter Soll-Flußraten Q₁ und Q₂ gesteuert, denen zwei vorbestimmte Drehzahlen n₁ und n₂ der Spülpumpe 14 zugeordnet sind. Die Steuerschaltung weist eine Steuereinheit 36 für das Arbeitsinstrument 23 auf, die über bspw. ein Relais 38 und Schalter 40 und 42 automatisch zwischen diesen beiden Soll-Flußraten Q₁ und Q₂ in Abhängigkeit des Betriebszustandes des bereits oben erwähnten, mit der zweiten Saugleitung 22 verbundenen Arbeitsinstruments 23 umschaltet. Wird das Arbeitsinstrument 23, beispielsweise ein Resektionsinstrument, eingeschaltet, stellt die Steuerschaltung automatisch die höhere Soll-Flußrate Q₁ entsprechend der höheren Drehzahl n₁ an der Spülpumpe 14 ein, und beim Ausschalten des Arbeitsinstruments schaltet die Steuerschaltung automatisch auf die niedrigere Soll-Flußrate Q₂ entsprechende der niedrigeren Drehzahl n₂ um, oder umgekehrt.

Des weiteren übernimmt die Steuerschaltung auch die automatische Umschaltung zwischen der Absaugung der Flüssigkeit aus dem Körperhohlraum 12 über die erste Saugleitung 20 oder die zweite Saugleitung 22, wenn das Arbeitsinstrument 23 eingeschaltet wurde, so daß dann die Flüssigkeit, ggf. nebst Körperflüssigkeiten und Gewebestücken über die zweite Saugleitung 22 durch das Arbeitsinstrument hindurch abgesaugt wird. An der ersten Saugleitung 20 und an der zweiten Saugleitung 22 sind in Fig. 1 schematisch von der Steuerschaltung gesteuerte Ventile 32 bzw. 34, bspw. Schlauchquetschventile, vorgesehen, die entsprechend über mit der Steuereinheit 36 für das Arbeitsinstrument 23 verbundene Schalter 44 und 46 geschlossen und geöffnet werden, um die Umschaltung zwischen den Saugleitungen 20 und 22 vorzunehmen.

Gemäß Fig. 2 weist die Steuerschaltung weiterhin einen Regler 48 auf, der eingangsseitig mit einem Subtrahierglied oder Vergleicher 50 verbunden ist, das eingangsseitig mit dem Signal der jeweils eingestellten Soll-Flußrate Q₁ oder Q₂ beaufschlagt wird, und ebenso mit dem Signal der Ist-Drehzahl nᵢₛₜ der Saugpumpe 24, die einer Ist-Flußrate Qᵢₛₜ entspricht, insbesondere wenn die Saugpumpe 24 wie in dem gezeigten Ausführungsbeispiel eine Rollenpumpe ist. Der Regler 48 ist ausgangsseitig mit der Spülpumpe 14 verbunden, und gibt auf diese ein Signal, das der Soll-Flußrate Qₛ entspricht.

Der Drucksensor 28 gibt ferner ein Signal auf ein weiteres Subtrahierglied oder Vergleicher 52, das dem Ist-Druck, der vom. Drucksensor 28 erfaßt wurde, entspricht. Das Subtrahierglied oder Vergleicher 52 ist weiterhin eingangsseitig mit einem Soll-Druckgeber verbunden, der auf das Subtrahierglied oder Vergleicher 52 ein dem Soll-Druck Pₛ entsprechendes Signal gibt. Das Subtrahierglied oder Vergleicher 52 ist ausgangsseitig mit einem weiteren Regler 54 verbunden, der ausgangsseitig mit der Saugpumpe 24 verbunden ist.

Nachfolgend wird nun die Steuerung bzw. Regelung der Vorrichtung 10 näher beschrieben.

Die Regelung der Saugpumpe 24 zur Aufrechterhaltung des Soll-Druckes pₛ im Körperhohlraum 12 in Abhängigkeit des vom Drucksensor 28 erfaßten Ist-Druckes p (unterbrochene Linie 29 in Fig. 1) erfolgt so, daß die Drehzahl der Saugpumpe 24 erhöht wird, wenn der Ist-Druck über den Soll-Druck Pₛ ansteigt, und die Drehzahl der Saugpumpe 24 wird entsprechend erniedrigt, wenn der Ist-Druck p unter den Soll-Druck Pₛ fällt.

Im Fall, daß die Spülpumpe 14 eine Zahnradpumpe ist, ist die Flußrate, die von der Spülpumpe 14 erzeugt wird, nicht eindeutig mit der Drehzahl der Spülpumpe 14 korreliert, wie es in der Regel bei einer Rollenpumpe der Fall ist. Die Flußrate hängt bei einer Zahnradpumpe sowohl von dem in der Spülleitung 16 herrschenden Spüldruck als auch von dem in der Saugleitung 20 bzw. 22 herrschenden Saugdruck ab. Aus diesem Grund ist es nicht möglich, die Flußrate allein mittels der Drehzahl der Spülpumpe 14 zu steuern, sondern es bedarf einer Regelung. Daher ist die Steuerschaltung insbesondere dann, wenn die Spülpumpe 14 eine Zahnradpumpe ist, so ausgestaltet, daß eine Rückkopplung der Drehzahl nᵢₛₜ auf die Spülpumpe 14 rückgekoppelt wird, d.h. die Steuerschaltung verwendet die Drehzahl nᵢₛₜ der Saugpumpe als Gebersignal für die Ist-Flußrate. Wenn nun die Flüssigkeit ungehindert durch den Körperhohlraum 12 strömt und keine Flüssigkeit in die Blutbahn gelangt, d.h. wenn die Flußrate in der Spülleitung 16 und in der Saugleitung 20 bzw. 22 gleich sind, ist eine unabhängige Einstellung der Flußrate und des Druckes ohne weiteres möglich.

Wird nun beispielsweise die Soll-Flußrate von Q₂ nach Q₁ erhöht, in deren Abhängigkeit wie bereits vorerwähnt die Spülpumpe 14 gesteuert ist, wird der Regler 48 die Drehzahl n der Spülpumpe 14 erhöhen, wodurch der Ist-Druck p in der Spülleitung 16 zunächst ansteigen würde. Dies würde dazu führen, daß die Saugpumpe 24 über den Regler 54 ihre Drehzahl nᵢₛₜ erhöht, um diesen erhöhten Ist-Druck p wieder abzubauen, und dies solange, bis die Flußrate in der Saugleitung 20 bzw. 22 an die neue vorgegebene Flußrate in der Spülleitung 16 angeglichen ist.

Wird andererseits der Soll-Druck pₛ erhöht, so würde der Regler 54 der Steuerschaltung zunächst die Saugpumpe 24 in ihrer Drehzahl nᵢₛₜ drosseln. Dies würde zu einer Regelabweichung bei der Spülpumpe 14 führen, die zunächst ihre Drehzahl n erhöht, was zu einem weiteren Anstieg des Ist-Drucks führt, was wiederum von der Saugpumpe 24 durch eine Erhöhung der Saugleistung zu kompensieren versucht würde. Auf diese Weise kann eine Erhöhung des Soll-Druckes pₛ erreicht werden, ohne daß die Soll-Flußrate Qs letzten Endes verändert wird.

Tritt jedoch beispielsweise eine Obstruktion in der Saugleitung 20 oder 22 auf, die den Abfluß der Flüssigkeit aus dem Körperhohlraum 12 behindern würde, tritt ein anderer Mechanismus in Kraft, der darauf beruht, daß dann, wenn allgemein die Drehzahl einer Rollenpumpe erhöht wird, ohne daß entsprechend Flüssigkeit in ausreichender Menge nachfließen kann, der Druck in der Saugleitung 20 bzw. 22 sinkt. Dies führt dazu, daß der Pumpenschlauch der Rollenpumpe im Saugbereich seinen vollen Querschnitt nicht erreicht, d.h. teilweise kollabiert, so daß die Flußrate trotz gestiegener Drehzahl nᵢₛₜ nicht im gleichen Maße zunimmt, gegebenenfalls sogar abnimmt. Dies bewirkt wiederum, daß die Spülpumpe 14, entsprechend der Drehzahl nᵢₛₜ der Saugpumpe 24, die für die Spülpumpe 14 ein Maß für die Ist-Flußrate Qᵢₛₜ darstellt, annehmen muß, daß die Ist-Flußrate Qᵢₛₜ zu hoch ist und dementsprechend ihre Drehzahl n reduziert. Dies führt zu der gewünschten Auswirkung, daß der Druck im Körperhohlraum 12 durch die immer geringer werdende Drehzahl n der Spülpumpe 14 wieder abgebaut bzw. nicht weiter aufgebaut wird.

Die vorstehend genannte Regelabhängigkeit funktioniert nicht nur dann, wenn als Spülpumpe 14 eine Zahnradpumpe eingesetzt wird, sondern auch dann, wenn als Spülpumpe 14 eine Rollenpumpe eingesetzt wird, obwohl eine Rollenpumpe eigentlich keiner Regelung bedarf, wie oben erwähnt wurde.

Des weiteren kann insbesondere dann, wenn sowohl die Spülpumpe 14 als auch die Saugpumpe 24 jeweils eine Rollenpumpe ist, der Verlust an Flüssigkeit im Körperhohlraum 12 durch eine Subtraktion der beiden Drehzahlen nᵢₛₜ der Saugpumpe 24 und der Drehzahl n der Spülpumpe 14 erfaßt werden.

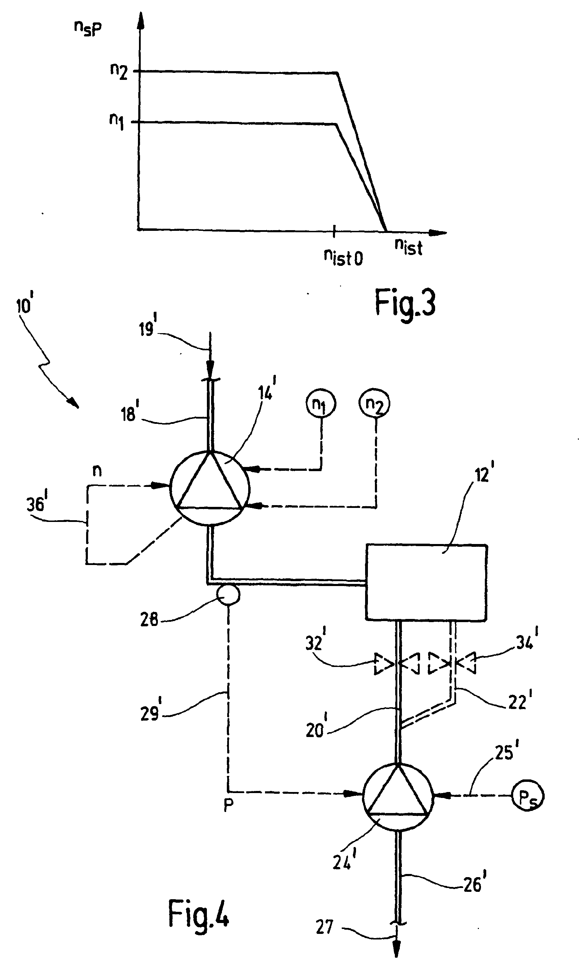

Eine geringfügig abgewandelte Regelung der Spülpumpe 14 in Abhängigkeit von der Saugpumpe 24 ist in Fig. 3 schematisch dargestellt. Kommt es beispielsweise zu einer Obstruktion in der ersten Saugleitung 20 oder der zweiten Saugleitung 22, steigt der Ist-Druck p im Körperhohlraum 12 an, da die Spülpumpe 14 weiterhin Spülflüssigkeit über die Spülleitung 16 in den Körperhohlraum 12 zuführt. Da die Saugpumpe 24 in Abhängigkeit des vorbestimmten Soll-Druckes Pₛ gesteuert ist, führt die Druckerhöhung im Körperhohlraum 12 zu einer höheren Saugleistung der Saugpumpe 24, die sich in einer höheren Drehzahl nᵢₛₜ der Saugpumpe 24 äußert. Bei leichteren Obstruktionen reicht die Erhöhung der Saugleistung der Saugpumpe 24 aus, um die Obstruktion, beispielsweise in der Saugleitung 20 oder Saugleitung 22 festhängendes Gewebe, abzusaugen. Wenn die Beseitigung der Obstruktion jedoch nicht möglich ist, wird die Drehzahl nᵢₛₜ der Saugpumpe 24 weiter steigen und einen vorbestimmten Schwellenwert nₛₐ₀ überschreiben. Die Überschreitung des vorbestimmten Schwellenwertes nᵢₛₜ₀ der Drehzahl nᵢₛₜ der Saugpumpe 24 führt dann über die Rückkopplung auf die Spülpumpe 14 dazu, daß die Drehzahl n bzw. die voreingestellte Drehzahl n₁ die voreingestellte Drehzahl n₂ erniedrigt wird, ggf. bis die Spülpumpe 14 auf die Drehzahl 0 gedrosselt wird.

Eine weitere Regelabhängigkeit der Spülpumpe kann in einer Steuerung der Spülpumpe in Abhängigkeit der Differenz der Drehzahlen von Saugpumpe und Spülpumpe bestehen.

In Fig. 4 ist eine Vorrichtung 10' zum Spülen eines Körperhohlraumes 12' mit einer Flüssigkeit dargestellt, die sich von der Vorrichtung 10' lediglich darin unterscheidet, daß die Spülpumpe 14' nicht in Abhängigkeit der Saugleistung der Saugpumpe 24' gesteuert ist. Die Spülpumpe 14' wird vielmehr lediglich in Abhängigkeit der vorbestimmten Soll-Flußrate bzw. zumindest zweier vorbestimmter Soll-Flußraten entsprechend zweier vorbestimmter Drehzahlen n₁ und n₂ so gesteuert, daß die vorbestimmten Soll-Flußraten im Körperhohlraum 12' aufrechterhalten werden; wie unter einer unterbrochenen Linie 36' angedeutet ist. Im übrigen unterscheidet sich die Vorrichtung 10' nicht von der Vorrichtung 10, so daß auf die Beschreibung der Vorrichtung 10. verwiesen werden kann, wobei gleiche Komponenten mit dem gleichen Bezugszeichen und einem hochgestellten ' versehen wurden.

In den zuvor beschriebenen Ausführungsbeispielen kann die Spülpumpe 14 bzw. die Spülpumpe 14' anstelle als Zahnradpumpe bzw. allgemein als Verdrängerpumpe auch als Zentrifugalpumpe ausgestaltet sein. Wenn eine derartige Zentrifugalpumpe als Spülpumpe verwendet wird, wird vorzugsweise zuvor die Korrelation zwischen der Flußrate und der Drehzahl der Zentrifugalpumpe ermittelt, und die Steuerschaltung greift dann auf diese Korrelation zurück, um die Soll-Flußrate einzustellen bzw. aufrechtzuerhalten.

## Patentansprüche

1. Vorrichtung zum Spülen eines Körperhohlraums (12; 12') mit einer Flüssigkeit, mit einer Spülpumpe (14; 14') und einer dieser zugeordneten zum Körperhohlraum hin führbaren Spülleitung (16; 16') zum Zuführen der Flüssigkeit in den Körperhohlraum (12; 12'), und mit zumindest einer von dem Körperhohlraum (12; 12') weg führbaren Saugleitung (20, 22; 20', 22') und einer dieser zugeordneten Saugpumpe (24; 24') zum Absaugen der Flüssigkeit von dem Körperhohlraum (12; 12'), und mit einer Steuerschaltung, die die Spülpumpe (14; 14') und die Saugpumpe (24; 24') derart steuert, daß die Flüssigkeit im wesentlichen mit einem vorbestimmten Soll-Druck und einer vorbestimmten Soll-Flußrate durch den Körperhohlraum (12; 12') tritt, wobei die Steuerschaltung die Spülpumpe (14; 14') in Abhängigkeit von der Soll-Flußrate steuert, während sie die Saugpumpe (24; 24') in Abhängigkeit des Soll-Drucks steuert, **dadurch** gekennzeich-net, daß die Steuerschaltung desweiteren derart ausgebildet ist, daß sie die Drehzahl der Saugpumpe (24) als Gebersignal verwendet und in Abhängigkeit dieses Gebersignals die Spülpumpe (14) steuert.

2. Vorrichtung nach Anspruch 1, **dadurch gekennzeichnet, daß** der Spülleitung ein Drucksensor (28; 28') zur Erfassung des Ist-Druckes zugeordnet ist, und daß die Steuerschaltung desweiteren derart ausgebildet ist, daß sie die Saugpumpe (24; 24') in Abhängigkeit des vom Drucksensor (28; 28') erfaßten Ist-Druckes zur Aufrechterhaltung des Soll-Druckes steuert.

3. Vorrichtung nach Anspruch 1 oder 2, **dadurch gekennzeichnet, daß** die Steuerschaltung derart ausgebildet ist, daß sie die Spülpumpe (14) in Abhängigkeit der Saugleistung der Saugpumpe (24) steuert, derart, daß bei einer Erhöhung der Saugleistung der Saugpumpe (24), zumindest, wenn diese über einen vorbestimmten Schwellenwert steigt, die Leistung der Spülpumpe (14) reduziert wird.

4. Vorrichtung nach einem der Ansprüche 1 bis 3, **dadurch gekennzeichnet, daß** die Spülpumpe (14; 14') und/oder die Saugpumpe (24; 24') jeweils Verdrängerpumpen sind.

5. Vorrichtung nach Anspruch 4, **dadurch gekennzeichnet, daß** die Spülpumpe (14; 14') eine Rollenpumpe oder eine Zahnradpumpe ist.

6. Vorrichtung nach Anspruch 4 oder 5, **dadurch gekennzeichnet, daß** die Saugpumpe (24; 24')eine Rollenpumpe ist.

7. Vorrichtung nach einem der Ansprüche 1 bis 3, **dadurch gekennzeichnet, daß** die Spülpumpe eine Zentrifugalpumpe ist.

8. Vorrichtung nach einem der Ansprüche 1 bis 7, **dadurch gekennzeichnet, daß** die Steuerschaltung derart ausgebildet ist, daß sie die Spülpumpe (14) zumindest bei Überschreiten eines Flußraten-Schwellenwertes in Abhängigkeit von der Drehzahl der Saugpumpe (24) steuert, derart, daß die Drehzahl der Spülpumpe (14) vermindert wird, wenn die Drehzahl der Saugpumpe (24) steigt.

9. Vorrichtung nach einem der Ansprüche 1 bis 8, **dadurch gekennzeichnet, daß** die Steuerschaltung derart ausgebildet ist, daß sie die Spülpumpe (14) in Abhängigkeit von der Differenz der Drehzahlen von Spülpumpe (14) und Saugpumpe (24) steuert.

10. Vorrichtung nach einem der Ansprüche 1 bis 9, **dadurch gekennzeichnet, daß** die Steuerschaltung derart ausgebildet ist, daß sie die Spülpumpe (14) in Abhängigkeit von zumindest zwei unterschiedlichen Soll-Flußraten (Q₁, Q₂) steuert.

11. Vorrichtung nach Anspruch 10, **dadurch gekennzeichnet, daß** die Steuerschaltung derart ausgebildet ist, daß sie die Spülpumpe (14) zwischen den zumindest zwei Soll-Flußraten (Q₁, Q₂) in Abhängigkeit von dem Betriebszustand eines mit der Steuerschaltung verbundenen Arbeitsinstruments (23) umschaltet.

12. Vorrichtung nach einem der Ansprüche 1 bis 11, **dadurch gekennzeichnet, daß** zumindest zwei parallel geschaltete Saugleitungen (20, 22; 20', 22') von dem Körperhohlraum (12; 12') wegführbar sind, wobei die Saugpumpe (24; 24') beiden Saugleitungen (20, 22; 20', 22') zugeordnet ist, wobei ferner eine Saugleitung (22; 22') mit einem Arbeitsinstrument (23) verbunden ist, und wobei zwischen den beiden Saugleitungen (20, 22; 20', 22') in Abhängigkeit von dem Betriebszustand des Arbeitsinstruments (23) umschaltbar ist.

13. Vorrichtung nach einem der Ansprüche 1 bis 12, **dadurch gekennzeichnet, daß** die Steuerschaltung derart ausgebildet ist, daß sie die Differenz zwischen den Drehzahlen der Saugpumpe (24) und der Spülpumpe (14) ermittelt.

## Claims

1. An apparatus for irrigating a body cavity (12; 12') with a liquid, comprising an irrigation pump (14; 14') and, associated therewith, an irrigation conduit (16; 16') which can be led to the body cavity (12; 12') for feeding the liquid into the body cavity (12; 12'), and at least one suction conduit (20, 22; 20', 22') which can be led away from the body cavity (12; 12') and, associated therewith, a suction pump (24; 24') for drawing off the liquid from the body cavity (12; 12'), and a control circuit, which controls the irrigation pump (14; 14') and the suction pump (24; 24') such that the liquid passes through the body cavity (12; 12') substantially with a predetermined nominal pressure and a predetermined nominal flow rate, wherein the control circuit controls the irrigation pump (14; 14') as a function of the nominal flow rate, while it controls the suction pump (24; 24') as a function of the nominal pressure, **characterized in that** the control circuit is further configured such that it uses the speed of the suction pump (24) as a control signal and controls the irrigation pump (14) as a function of said control signal.

2. The apparatus of claim 1, **characterized in that** a pressure sensor (28; 28') is associated with the irrigation conduit for determining the actual pressure, and the control circuit is configured such that it controls the suction pump (24; 24') as a function of the actual pressure determined by the pressure sensor (28; 28') for maintaining the nominal pressure.

3. The apparatus of claim 1 or 2, **characterized in that** the control circuit is configured such that it controls the irrigation pump (14) as a function of the suction power of the suction pump (24) such that the power of the irrigation pump (14) is reduced when the suction power of the suction pump (24) is increased, at least when the suction power of the suction pump (24) exceeds a predetermined threshold value.

4. The apparatus of anyone of claims 1 through 3, **characterized in that** the irrigation pump (14; 14') and/or the suction pump (24; 24') are displacement pumps.

5. The apparatus of claim 4, **characterized in that** the irrigation pump (14; 14') is a roller pump or a gear pump.

6. The apparatus of claim 4 or 5, **characterized in that** the suction pump (24; 24') is a roller pump.

7. The apparatus of anyone of claims 1 through 3, **characterized in that** the irrigation pump is a centrifugal pump.

8. The apparatus of anyone of claims 1 through 7, **characterized in that** the control circuit is configured such that it controls the irrigation pump (14) as a function of the speed of the suction pump (24), at least when a flow rate threshold value is exceeded, such that the speed of the irrigation pump (14) is reduced when the speed of the suction pump (24) increases.

9. The apparatus of anyone of claims 1 through 8, **characterized in that** the control circuit is configured such that it controls the irrigation pump (14) as a function of the difference between the speeds of the irrigation pump (14) and the suction pump (24).

10. The apparatus of anyone of claims 1 through 9, **characterized in that** the control circuit is configured controls the irrigation pump (14) as a function of at least two different nominal flow rates (Q₁, Q₂).

11. The apparatus of claim 10, **characterized in that** the control circuit is configured such that it switches over the irrigation pump (14) between the at least two nominal flow rates (Q₁, Q₂) as a function of the operating condition of a working instrument (23) connected with the control circuit.

12. The apparatus of anyone of claims 1 through 11, **characterized in that** at least two parallel suction conduits (20, 22; 20', 22') can be led away from the body cavity (12; 12'), wherein the suction pump (24; 24') is associated with both suction conduits (20, 22; 20', 22'), wherein further one suction conduit (22; 22') is connected with a working instrument (23), and wherein it can be switched over between both suction conduits (20, 22; 20', 22') as a function of the operating condition of the working instrument (23).

13. The apparatus of anyone of claims 1 through 12, **characterized in that** the control circuit is configured such that it determines the difference between the speeds of the suction pump (24) and the irrigation pump (14).

## Revendications

1. Dispositif pour l'irrigation d'une cavité corporelle (12 ; 12') avec un liquide, comprenant une pompe d'irrigation (14 ; 14') et une conduite d'irrigation (16 ; 16') branchée sur cette pompe et pouvant être guidée en direction de la cavité corporelle pour l'arrivée du liquide dans la cavité corporelle (12 ; 12'), et au moins une conduite d'aspiration (20, 22 ; 20', 22') pouvant être guidée à partir de la cavité corporelle et une pompe d'aspiration (24 ; 24') branchée sur cette conduite pour l'aspiration du liquide de la cavité corporelle (12 ; 12'), et un circuit de commande, qui commande la pompe d'irrigation (14 ; 14') et la pompe d'aspiration (24 ; 24'), de sorte que le liquide circule sensiblement avec une pression théorique prédéfinie et un débit théorique prédéfini à travers la cavité corporelle (12 ; 12'), le circuit de commande commandant la pompe d'irrigation (14 ; 14') en fonction du débit théorique, alors qu'il commande la pompe d'aspiration (24 ; 24') en fonction de la pression théorique, **caractérisé en ce que** le circuit de commande est agencé également de telle sorte qu'il utilise le régime de la pompe d'aspiration (24) comme signal d'entrée et commande la pompe d'irrigation (14) en fonction de ce signal d'entrée.

2. Dispositif selon la revendication 1, **caractérisé en ce que** à la conduite d'irrigation est branchée sur capteur de pression (28 ; 28'), pour l'enregistrement de la pression réelle, et **en ce que** le circuit de commande (1) est agencé également de telle sorte qu'il commande la pompe d'aspiration (24 ; 24') en fonction de la pression réelle enregistrée par le capteur de pression (28 ; 28') pour le maintien de la pression théorique.

3. Dispositif selon la revendication 1 ou 2, **caractérisé en ce que** le circuit de commande est agencé de telle sorte qu'il commande la pompe d'irrigation (14) en fonction du débit d'aspiration de la pompe d'aspiration (24), de sorte que, dans le cas d'une élévation de la puissance d'aspiration de la pompe d'aspiration (24), au moins lorsque celle-ci excède une valeur de seuil prédéfinie, la puissance de la pompe d'irrigation (14) se trouve réduite.

4. Dispositif selon l'une quelconque des revendications 1 à 3, **caractérisé en ce que** la pompe d'irrigation (14 ; 14'), et/ou la pompe d'aspiration (24 ; 24') sont respectivement des pompes volumétriques.

5. Dispositif selon la revendication 4, **caractérisé en ce que** la pompe d'irrigation (14 ; 14') est une pompe à rouleaux ou une pompe à engrenages.

6. Dispositif selon la revendication 4 ou 5, **caractérisé en ce que** la pompe d'aspiration (24 ; 24') est une pompe à rouleaux.

7. Dispositif selon l'une quelconque des revendications 1 à 3, **caractérisé en ce que** la pompe d'irrigation est une pompe centrifuge.

8. Dispositif selon l'une quelconque des revendications 1 à 7, **caractérisé en ce que** le circuit de commande est agencé de telle sorte qu'il commande la pompe d'irrigation (14), au moins en cas de dépassement d'une valeur seuil de débit, en fonction du régime de la pompe d'aspiration (24), de sorte que le régime de la pompe d'irrigation (14) est réduit lorsque le régime de la pompe d'aspiration (24) augmente.

9. Dispositif selon l'une quelconque des revendications 1 à 8, **caractérisé en ce que** le circuit de commande est agencé de telle sorte qu'il commande la pompe d'irrigation (14) en fonction de la différence des régimes de la pompe d'irrigation (14) et de la pompe d'aspiration (24).

10. Dispositif selon l'une quelconque des revendications 1 à 9, **caractérisé en ce que** le circuit de commande est agencé de telle sorte qu'il commande la pompe d'irrigation (14) en fonction d'au moins deux débits théoriques (Q₁, Q₂) différents.

11. Dispositif selon la revendication 10, **caractérisé en ce que** le circuit de commande est agencé de telle sorte qu'il commute la pompe d'irrigation (14) entre au moins les deux débits théoriques (Q₁, Q₂), en fonction de l'état de service d'un instrument de travail (23) relié au circuit de commande.

12. Dispositif selon l'une quelconque des revendications 1 à 11, **caractérisé en ce qu'**au moins deux conduites d'aspiration (20, 22 ; 20', 22') branchées en parallèle peuvent être guidées à partir de la cavité corporelle (12 ; 12'), la pompe d'aspiration (24 ; 24') étant branchée aux deux conduites d'aspiration (20, 22 ; 20', 22'), **en ce qu'**au moins une conduite d'aspiration (22 ; 22') est reliée à un instrument de travail (23), et une commutation est effectuée entre les deux conduites d'aspiration (20, 22 ; 20', 22') en fonction de l'état de service de l'instrument de travail (23).

13. Dispositif selon l'une quelconque des revendications 1 à 12, **caractérisé en ce que** le circuit de commande est agencé de telle sorte qu'il détermine la différence entre les régimes de la pompe d'aspiration (24) et de la pompe d'irrigation (14).
